# EUROPEAN PATENT APPLICATION

(11) **EP 4 810 025 A1**
(43) Date of publication of application: **23.09.2026**
(21) Application number: 25210410.4
(22) Date of filing: 22.10.2025
(51) Int. Cl.: A61K 9/20, A61K 9/28, A61K 31/165, A61P 25/24

(54) **A FILM COATED TABLET FORMULATION OF LISDEXAMFETAMINE DIMESILATE**

(30) Priority: 20.03.2025 TR 2025003337
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); SARP, Onder, Istanbul (TR); AKYEL, Fehimenur, Istanbul (TR)
(74) Representative: Genç Ilhan, Oznur

(57) **Abstract**

The present invention relates to a film coated tablet formulation comprising lisdexamfetamine dimesylate and at least one stabilizer. Furthermore, the invention discloses a process which is simple, rapid, cost effective, time-saving and industrially convenient process.

## Description

### Field of the Invention

The present invention relates to a film coated tablet formulation comprising lisdexamfetamine dimesylate and at least one stabilizer. Furthermore, the invention discloses a process which is simple, rapid, cost effective, time-saving and industrially convenient process.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate is also referred to as I-lysine-d-amphetamine dimesylate of Formula I is chemically named as (2S)-2,6-Diamino-N-[(1S)-1-methyl-2-phenylethyl]-hexanamide dimesylate.

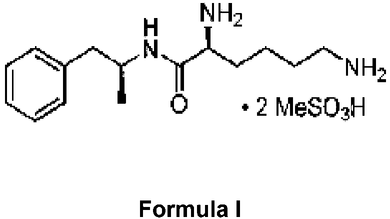

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50, 60 and 70 mg hard gelatine capsules. Each capsules contains 10, 20, 30, 40, 50, 60 and 70 mg lisdexamfetamine dimesylate, equivalent to 5.8 mg, 11.6 mg, 17.3 mg, 23.1 mg, 28.9 mg, 34.7 mg, 40.5 mg lisdexamfetamine, respectively.

The patent WO2006121552A2 discloses a capsule and chewable tablet formulation containing lisdexamfetamine dimesylate, microcrystalline cellulose, croscarmellose sodium, magnesium stearate.

In the prior art have described the capsule and chewable tablet formulations of lisdexamfetamine. The possibility of a film coated tablet form has been mentioned, but no example formulation has been provided.

Lisdexamfetamine dimesylate is an active substance that is deliquescent and needs to be formulated accordingly. Therefore, a formulation that can increase the stability of the formulation by providing moisture balance is needed. We obtained a formulation with high stability and desired dissolution profile by using lisdexamfetamine dimesylate and a suitable stabilizer.

### Detailed Description of the Invention

The main object of the present invention is to provide a film coated tablet formulation comprising lisdexamfetamine dimesylate having the high stability and desired dissolution profile.

Another object of the present invention is to provide a process for a film coated tablet formulation comprising lisdexamfetamine dimesylate prepared by simple, easy, time-saving and industrially convenient process.

Lisdexamfetamine dimesylate formulation is known in the prior art, but there is still a need for a formulation with high stability and dissolution profile. Lisdexamfetamine dimesylate is a deliquescent active substance. It is therefore important to ensure the stability of the formulation. In order to increase the stability of lisdexamfetamine dimesylate formulation, film-coated tablet form and at least one stabilizer were used. Film-coated tablet formulation was preferred because it protects the formulation content from external factors (moisture, light etc.). Since lisdexamfetamine dimesylate is affected by moisture, when a suitable stabilizer is used, the stabilizer helps to increase the stability of the formulation by regulating the moisture balance. It also helps to distribute lisdexamfetamine dimesylate more homogeneously in the tablet and provides uniform distribution and hence content uniformity.

According to this embodiment of the invention, a film coated tablet formulation comprising lisdexamfetamine dimesylate and at least one stabilizer.

According to this embodiment of the present invention, the amount of the lisdexamfetamine dimesylate is between 5.0% and 30.0% by weight of the total formulation. Preferably, it is between 10.0% and 25.0% by weight of the total formulation.

Suitable stabilizers are selected from the group comprising magnesium aluminometasilicate, glycerol dibehenate, isomalt, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

According to this embodiment of the present invention, the stabilizer is magnesium aluminometasilicate and glycerol dibehenate or mixture thereof. When these were used, thus obtaining a formulation with high stability.

According to this embodiment of the present invention, the stabilizer is magnesium aluminometasilicate.

According to this embodiment of the present invention, the stabilizer is glycerol dibehenate. It is also known that glyceryl dibehenate is used as a moisture protective agent.

According to this embodiment of the present invention, the amount of stabilizer is between 0.5% and 20.0% by weight of the total formulation. Preferably, it is between 2.0% and 15.0% by weight in the total formulation.

According to this embodiment of the present invention, the film coated tablet formulation further comprises at least one pharmaceutically acceptable excipient selected from the group comprising fillers, disintegrants, binders, glidants, lubricants or mixtures thereof.

Suitable fillers are selected from the group comprising microcrystalline cellulose, dibasic calcium phosphate, lactose, starch, sucrose, ammonium alginate, calcium phosphate, neutral pellets, calcium sulfate, cellulose acetate, erythritol, ethyl cellulose, fructose, glyceryl palmitostearate, lactose, mannitol, magnesium carbonate, magnesium oxide, maltodextrin, maltose, medium chain triglycerides, polydextrose, polymethacrylates, sodium chloride, sorbitol, sugar spheres, tragacanth, trehalose, polysorbate 80, xylitol or mixtures thereof.

According to this embodiment of the present invention, the filler is microcrystalline cellulose or dibasic calcium phosphate or mixture thereof.

According to one embodiment of the present invention, the amount of the filler is between 55.0% and 85.0% by weight in the total formulation. Preferably, it is between 60.0% and 80.0% by weight of the total formulation.

In addition, the moisture content of microcrystalline cellulose was used below 1.5%. In this way, it helped to increase the stability and content uniformity of lisdexamfetamine dimesylate, which is sensitive to moisture.

Suitable disintegrants are selected from the group comprising sodium starch glycolate, croscarmellose sodium, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to one embodiment of the present invention, the disintegrant is sodium starch glycolate or croscarmellose sodium or crospovidone or low-substituted hydroxypropyl cellulose or mixture thereof.

According to one embodiment of the present invention, the amount of disintegrant is between 0.5% and 15.0% by weight of the total formulation. Preferably, it is between 1.0% and 10.0% by weight of the total formulation.

Suitable binders are selected from the group comprising povidone, hydroxypropyl cellulose, carboxymethylcellulose sodium, carboxymethylcellulose calcium, cellulose acetate phthalate, starch, corn starch, pregelatinized starch, ethyl cellulose, glyceryl behenate, hydrogenated vegetable oil type I, hydroxyethyl cellulose, hydroxyethyl methyl cellulose, hydroxypropyl starch, hydroxypropyl methyl cellulose, polyoxyethylene-alkyl ethers or mixtures thereof.

According to one embodiment of the present invention, the binder is povidone or hydroxypropyl cellulose or mixtures thereof.

According to one embodiment of the present invention, the amount of the binder is between 0.1% and 10.0% by weight in the total formulation. Preferably, it is between 1.0% and 5.0% by weight of the total formulation. Also, this ratio helps to provide desired release profile.

Suitable lubricants are selected from the group comprising magnesium stearate, sodium stearyl fumarate, talc, calcium stearate, zinc stearate, magnesium lauryl sulfate, sodium oleate, sodium acetate, stearic acid, fumaric acid, or mixtures thereof.

According to one embodiment of the present invention, the lubricant is magnesium stearate.

According to one embodiment of the present invention, the amount of lubricant is between 0.01% and 5.0% by weight in the total formulation.

Suitable glidants are selected from the group comprising colloidal silicon dioxide, aluminum silicate, starch or mixtures thereof.

According to one embodiment of the present invention, the glidant is colloidal silicon dioxide.

According to one embodiment of the present invention, the amount of glidant is between 0.01% and 10.0% by weight in the total formulation.

According to this embodiment of the invention, a process for a film coated tablet formulations comprises the following steps:
a. Sieving and mixing lisdexamfetamine dimesylate and at least one stabilizer,
b. Sieving at least one filler and at least one disintegrant and at least one binder and at least one glidants/lubricants and mixing them with the powder mixture obtained in (a),
c. Compressing the resulting final mixture into tablets,
d. Coating of compressed tablets.

### Example 1;

| **Ingredients** | **%by weight** | **%by weight** |
|---|---|---|
| Lisdexamfetamine Dimesylate | 14.3 | 5-25 |
| Microcrystalline Cellulose | 72.4 | 60-80 |
| Povidone | 3.1 | 0.5-10 |
| Crospovidone | 4.1 | 0.5-10 |
| Glycerol Dibehenate | 5.1 | 1-10 |
| Colloidal Silicon Dioxide | 1.0 | 0.1-10 |
| Core Tablet Weight | **100** | **100** |

| **Opadry AMB II** | | |
|---|---|---|
| *Polyvinyl Alcohol - Part. Hydrolyzed | 3.1 | 1-5 |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | - | - |

A process for example 1;
a) Sieving and mixing lisdexamfetamine dimesylate and 80% of glycerol dibehenate,
b) Sieving microcrystalline cellulose PH-112, povidone, 50% of crospovidone Cl, and 50% of colloidal silicon dioxide, and adding to the mixture and mixing,
c) Compaction and sieving of the resulting mixture,
d) Sieving 20% of glycerol dibehenate, 50% of crospovidone Cl, and 50% of colloidal silicon dioxide, and adding to the mixture and mixing,
e) Compressing the resulting final mixture into tablets,
f) Coating of compressed tablets.

### Example 2;

| **Ingredients** | **%by weight** | **%by weight** |
|---|---|---|
| Lisdexamfetamine Dimesylate | 20 | 10-30 |
| Microcrystalline Cellulose | 64.3 | 55-75 |
| Hydroxypropyl Cellulose | 2.0 | 0.1-10 |
| Low Substituted Hydroxypropyl Cellulose | 8.5 | 1-15 |
| Glycerol Dibehenate | 4.3 | 0.5-10 |
| Colloidal Silicon Dioxide | 0.9 | 0.01-10 |
| **Core Tablet Weight** | **100** | **100** |

| **Opadry AMB II** | | |
|---|---|---|
| *Polyvinyl Alcohol - Part. Hydrolyzed | 2.9 | 1-5 |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | - | - |

A process for example 2;
a) Sieving and mixing lisdexamfetamine dimesylate and 80% of glycerol dibehenate,
b) Sieving microcrystalline cellulose, hydroxypropyl cellulose, 50% of low substituted hydroxypropyl cellulose and 50% of colloidal silicon dioxide, and adding to the mixture and mixing,
c) Compaction and sieving of the resulting mixture,
d) Sieving 20% of glycerol dibehenate, 50% of low substituted hydroxypropyl cellulose and 50% of colloidal silicon dioxide, and adding to the mixture and mixing,
e) Compressing the resulting final mixture into tablets,
f) Coating of compressed tablets.

### Example 3;

| **Ingredients** | **%by weight** | **%by weight** |
|---|---|---|
| Lisdexamfetamine Dimesylate | 14.3 | 5-25 |
| Microcrystalline Cellulose PH-112 | 46.9 | 35-52 |
| Dibasic Calcium Phosphate Anhydrous | 28.2 | 15-33 |
| Povidone | 2.0 | 0.1-10 |
| Sodium Starch Glycolate | 4.3 | 0.5-10 |
| Glycerol Dibehenate | 4.1 | 0.5-10 |
| Colloidal Silicon Dioxide | 0.2 | 0.01-5 |
| **Core Tablet Weight** | **100** | **100** |

| **Opadry AMB II** | | |
|---|---|---|
| *Polyvinyl Alcohol - Part. Hydrolyzed | 3.1 | 1-5 |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | | |

A process for example 3;
a) Sieving and mixing lisdexamfetamine dimesylate, 20% of microcrystalline cellulose and 80% of glycerol dibehenate,
b) Sieving dibasic calcium phosphate anhydrous, 80% of microcrystalline cellulose PH-112, povidone, and sodium starch glycolate, and adding to the mixture and mixing
c) Sieving colloidal silicon dioxide and adding it to the powder mixture and mixing,
d) Sieving 20% of glycerol dibehenate and adding it to the powder mixture and mixing,
e) Compressing the resulting final mixture into tablets,
f) Coating of compressed tablets.

### Example 4;

| **Ingredients** | **%by weight** | **%by weight** |
|---|---|---|
| Lisdexamfetamine Dimesylate | 20 | 10-30 |
| Microcrystalline Cellulose PH-112 | 63.7 | 55-75 |
| Povidone | 2.3 | 0.1-10 |
| Croscarmellose Sodium | 3.7 | 0.5-10 |
| Magnesium Aluminometasilicate | 10 | 1-20 |
| Colloidal Silicon Dioxide | 0.3 | 0.01-5 |
| Magnesium Stearate | 0.6 | 0.01-5 |
| **Core Tablet Weight** | **100** | **100** |

| **Opadry AMB II** | | |
|---|---|---|
| *Polyvinyl Alcohol - Part. Hydrolyzed | 2 | 1-5 |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | | |

A process for example 4;
a) Sieving and mixing lisdexamfetamine dimesylate and magnesium aluminometasilicate,
b) Sieving microcrystalline cellulose PH-112, povidone, and croscarmellose sodium, and adding to the mixture and mixing
c) Sieving colloidal silicon dioxide and adding it to the powder mixture and mixing,
d) Sieving magnesium stearate and adding it to the powder mixture and mixing,
e) Compressing the resulting final mixture into tablets,
f) Coating of compressed tablets.

## Claims

1. A film coated tablet formulation comprising lisdexamfetamine dimesylate and at least one stabilizer.

2. The film coated tablet formulation according to claim 1, wherein the amount of the lisdexamfetamine dimesylate is between 5.0% and 30.0% by weight of the total formulation.

3. The film coated tablet formulation according to claim 1, wherein stabilizers are selected from the group comprising magnesium aluminometasilicate, glycerol dibehenate, isomalt, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

4. The film coated tablet formulation according to claim 1 or 3, wherein the stabilizer is magnesium aluminometasilicate and glycerol dibehenate or mixture thereof.

5. The film coated tablet formulation according to claim 4, wherein the amount of stabilizer is between 0.5% and 20.0% by weight of the total formulation.

6. The film coated tablet formulation according to claim 1, wherein the film coated tablet formulation further comprises at least one pharmaceutically acceptable excipient selected from the group comprising fillers, disintegrants, binders, glidants, lubricants or mixtures thereof.

7. The film coated tablet formulation according to claim 6, wherein fillers are selected from the group comprising microcrystalline cellulose, dibasic calcium phosphate, lactose, starch, sucrose, ammonium alginate, calcium phosphate, neutral pellets, calcium sulfate, cellulose acetate, erythritol, ethyl cellulose, fructose, glyceryl palmitostearate, lactose, mannitol, magnesium carbonate, magnesium oxide, maltodextrin, maltose, medium chain triglycerides, polydextrose, polymethacrylates, sodium chloride, sorbitol, sugar spheres, tragacanth, trehalose, polysorbate 80, xylitol or mixtures thereof.

8. The film coated tablet formulation according to claim 6 or 7, wherein the filler is microcrystalline cellulose or dibasic calcium phosphate or mixture thereof.

9. The film coated tablet formulation according to claim 7 or 8, wherein the amount of the filler is between 55.0% and 85.0% by weight in the total formulation.

10. The film coated tablet formulation according to claim 6, wherein disintegrants are selected from the group comprising sodium starch glycolate, croscarmellose sodium, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

11. The film coated tablet formulation according to claim 6 or 10, wherein the disintegrant is sodium starch glycolate or croscarmellose sodium or crospovidone or low-substituted hydroxypropyl cellulose or mixture thereof.

12. The film coated tablet formulation according to claim 11, wherein the amount of disintegrant is between 0.5% and 15.0% by weight of the total formulation.

13. The film coated tablet formulation according to claim 6, wherein binders are selected from the group comprising povidone, hydroxypropyl cellulose, carboxymethylcellulose sodium, carboxymethylcellulose calcium, cellulose acetate phthalate, starch, corn starch, pregelatinized starch, ethyl cellulose, glyceryl behenate, hydrogenated vegetable oil type I, hydroxyethyl cellulose, hydroxyethyl methyl cellulose, hydroxypropyl starch, hydroxypropyl methyl cellulose, poly oxyethylene-alkyl ethers or mixtures thereof.

14. The film coated tablet formulation according to claim 6 or 13, wherein the binder is povidone or hydroxypropyl cellulose or mixtures thereof.

15. The film coated tablet formulation according to claim 14, wherein the amount of the binder is between 0.1% and 10.0% by weight in the total formulation.
